# EUROPEAN PATENT APPLICATION

(11) **EP 2 520 649 A2**
(43) Date of publication of application: **07.11.2012**
(21) Application number: 12174714.1
(22) Date of filing: 24.11.2008
(51) Int. Cl.: C12N 15/09, C07K 14/00, C12Q 1/68, C12N 15/113

(54) **MicroRNA antisense PNAs, compositions comprising the same, and methods for using and evaluating the same**

(30) Priority: 23.11.2007 KR 20070120459; 24.11.2008 KR 20080116856
(62) Divisional of application: 08851799.0
(71) Applicant: Panagene, Inc., Daejeon 305-804 (KR)
(72) Inventor: PARK, Hee Kyung, Daejeon 305-759 (KR); OH, Su Young, Daejeon 305-503 (KR)
(74) Representative: Hiebl, Inge Elisabeth

(57) **Abstract**

Disclosed are a microRNA antisense PNA capable of inhibiting the activity or function of microRNA, a composition for inhibiting the activity or function of microRNA containing the same, a method for inhibiting the activity or function of microRNA using the same, and a method for evaluating the effectiveness thereof.

## Description

### [Technical Field]

The present invention relates to a microRNA antisense PNA, a composition containing the same, and a method for using and evaluating the same, and more specifically, to a microRNA antisense PNA capable of inhibiting the activity or function of microRNA, also known as siRNA (small interfering RNA), a composition for inhibiting the activity or function of microRNA comprising the same, a method for inhibiting the activity or function of microRNA using the same, and a method for evaluating the same.

### [Background Art]

In 1993, some genes were found in *Caenorhabditis elegans* to regulate its developmental stages, among which let-7 and lin-4 were identified as small RNA fragments not translated into protein (non-coding RNA). These RNAs were commonly known as stRNA (small temporal RNA) because they are expressed in a specific developmental stage to regulate development. MicroRNA is a single-stranded RNA molecule of 21-25 nucleotides, which regulates gene expression in eukaryotes. Specifically, it is known to bind to 3' UTR (untranslated region) of mRNA for a specific gene to inhibit its translation. All the animal microRNAs studied heretofore decrease protein expression without affecting the level of mRNA for a specific gene.

MicroRNA is attached to RISC (RNA-induced silencing complex) to complementarily bind with a specific mRNA, but the center of microRNA remains mismatched, so it does not degrade mRNA, unlike conventional siRNAs. Unlike animal microRNAs, plant microRNAs perfectly match target mRNA to induce its degradation, which is referred to as "RNA interference."

Several plant microRNAs are involved in the translational regulation like animal microRNAs. Another report presents evidences that microRNAs induce methylation of chromatin in yeasts, including animals and plants, and so are involved in the transcriptional inhibition. Some of microRNAs are highly conserved inter-specifically, suggesting that they might be involved in important biological phenomena.

MicroRNA is produced through a two-step process. First, primary miRNA (pri-miRNA) is converted to pre-miRNA having step-loop structure of 70-90 nucleotides by an enzyme of RNase III type, Drosha, in a nucleus. Then, pre-miRNA is transported into cytoplasm and cleaved by an enzyme, Dicer, finally to form mature microRNA of 21-25 nucleotides. Recently, many researches have shown that microRNA plays an important role in cancer cells and stem cells as well as in cell proliferation, cell differentiation, apoptosis and control of lipid metabolism. However, many of microRNA functions remain unknown, for which studies are actively ongoing.

Researches on microRNA have been performed by investigating expression patterns by reporter gene analysis, microarray, northern blotting, and real-time polymerase chain reaction, or using antisense DNA or RNA (Boutla A, Delidakis C, and Tabler M. (2003) Developmental defects by antisense-mediated inactivation of micro-RNAs 2 and 13 in Drosophila and the identification of putative target genes. Nucleic Acids Res. 31(17): 4973-4980). Recently, 2'-O-Me RNA having higher binding affinity to RNA owing to its methyl group and having higher stability against nucleases than RNA itself, or 2'-O-methoxy oligonucleotide having even higher binding affinity than 2'-O-Me oligonucleotide have been synthesized and used as antisense against microRNA (Weiler J, Hunziker J and Hall J. (2006) Anti-miRNA oligonucleotides (AMOs): ammunition to target miRNAs implicated in human disease? Gene Therapy 13:496-502). To solve the drawback of DNA that it is readily degradable by nucleases, an oligonucleotide prepared by mixing LNA (Locked Nucleic Acid) and DNA has been used.

This oligonucleotide is known to have higher sensitivity and selectivity than DNA (Cha J A, Krichevsky A M and Kosik K S. (2005) microRNA-21 is an antiapoptotic factor in human glioblastoma cells. Cancer Res. 65:6029-6033). In addition, RNA antagomir having the attached cholesterol has also been synthesized to investigate functions of microRNA (Krutzfeldt J, Rajewsky N, Braich R, Rajeev K G, Tuschl T, Manoharan M and Stoffel M. (2005) Silencing of microRNAs in vivo with 'antagomirs'. Nature 438:685-689). They are antisense against microRNA that interrupt functions of microRNA, and so are extremely important for studies on functions of microRNA.

As described above, to overcome the drawbacks of DNA and RNA, such chemically modified oligonucleotides as LNA and 2-O-methyl oligonucleotide have been used but they are still degraded by endo- or exo-nucleases in cells, or have decreased specificity or cause cytotoxicity due to their modified structures (Crinelli R, Bianchi M, Gentilini L, and Magnani M. (2002) Design and characterization of decoy oligonucleotides containing locked nucleic acids. Nucleic Acids Res. 30(11):2435-2443; Hutvágner G, Simard MJ, Mello C C, Zamore P D. Hutvágner G, Simard M J, Mello C C, and Zamore P D. (2004) Sequence-specific inhibition of small RNA function. PLoS Biol. 2(4):E98). Therefore, there has been an eager demand on more efficient antisense oligonucleotides to interrupt functions of microRNA.

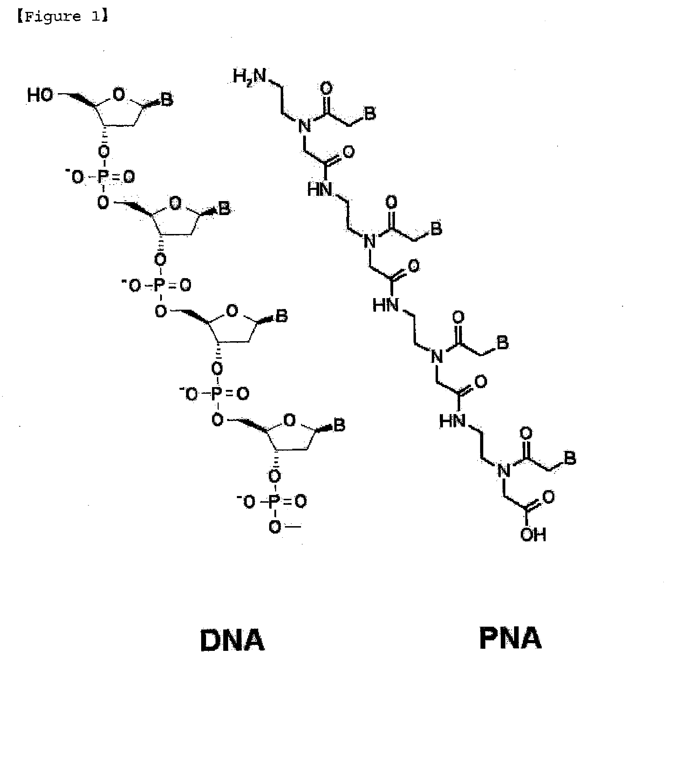

PNA (peptide nucleic acid) is a polymeric compound having the similar structure to DNA, which is a nucleic acid in the form of protein, capable of binding with DNA and RNA (Nielsen P E, Buchardt O, Egholm M, Berg R H, US Patent 5,539,082, Peptide nucleic acids). The backbone of PNA has the structure of polypeptide (Figure 1). While DNA has negative charge by its phosphate groups, PNA is electrically neutral by its peptide bonds. The conventional nucleases cannot recognize PNA, so PNA is not degraded by nucleases to have high stability *in vivo.* PNA has many advantages, that is, it has high binding affinity with DNA and RNA, is feasible for attachment of fluorophores or ions to enhance its solubility, has such a high specificity that even only one nucleotide difference can be detected from a whole genome, and can be modified to have another function by introducing a peptide thereto. Based on the above advantages, PNA can be applied for detection of mutations causing genetic disorders, or for early diagnosis of pathogenic bacterial and viral infection, and so widely applied in studies of cancer cell suppression, and in the fields of pathogenic microbiology, virology, etc. For the last several years, studies have been actively performed to develop PNA for antisense. However, there has been no attempt to use PNA as antisense against microRNA.

### [Disclosure]

### [Technical Problem]

To overcome the above described problems of the prior arts, the present inventors have conducted extensive studies to construct an antisense capable of specifically binding with microRNA, thereby inhibiting activity or function thereof, by using PNA having the above mentioned advantages. As a result, the present inventors developed an antisense PNA having superior and sustainable effect in cells, as compared with the conventional antisense DNA and RNA.

It is therefore an object of the present invention to provide a microRNA antisense PNA complementarily binding with microRNA, thereby inhibiting the activity or function thereof.

It is another object of the present invention to provide a composition for inhibiting activity or function of microRNA, containing the microRNA antisense PNA as an active ingredient.

It is still another object of the present invention to provide a method for inhibiting activity or function of microRNA by using the microRNA antisense PNA.

It is further still another object of the present invention to provide a method for evaluating the effectiveness of the microRNA antisense PNA.

### [Technical Solution]

It is a first aspect of the present invention to provide a microRNA antisense PNA, which consists of 10 to 25 nucleotides, and is capable of complementarily binding with microRNA, thereby inhibiting activity or function thereof.

It is a second aspect of the present invention to provide a composition for inhibiting activity or function of microRNA, containing the microRNA antisense PNA as an active ingredient.

It is a third aspect of the present invention to provide a method for inhibiting activity or function of microRNA, comprising the step of introducing into cells the microRNA antisense PNA.

It is a fourth aspect of the present invention to provide a method for evaluating the effectiveness of microRNA antisense PNA, comprising the step of measuring and comparing the expressions of microRNA, in presence and absence of the microRNA antisense PNA.

### [Description of Drawings]

The above and other objects, features and advantages of the present invention will become apparent from the following description of preferred embodiments given in conjunction with the accompanying drawings, in which:
Figure 1 shows the difference of the basic structure of DNA and PNA;
Figure 2 schematically shows the structure of a vector for cloning the binding sequence for target microRNA;
Figure 3 is a set of graphs comparing effects of antisense PNAs linked with K peptide (upper) and modified Tat peptide, R peptide (lower);
Figure 4 is a graph showing the effect of modified Tat peptide, R peptide, on the intracellular introduction of the antisense PNA;
Figure 5 is a graph comparing the effects of the conventional antisense and the antisense PNA on the target miR16;
Figure 6 is a graph showing the effects of the antisense PNA on the target miR16 at various concentrations;
Figure 7 is a graph comparing the effects of the antisense PNA on the target miR16 with the lapse of time;
Figure 8 is a graph comparing the effects of the conventional antisense and the antisense PNA on the target miR221;
Figure 9 is a graph comparing the effects of the conventional antisense and the antisense PNA on the target miR222;
Figure 10 is a graph showing the effect of the antisense PNA on the target miR31;
Figure 11 is a graph showing the effect of the antisense PNA on the target miR24;
Figure 12 is a graph showing the effect of the antisense PNA on the target miR21;
Figure 13 is a graph showing the effect of the antisense PNA on the target miR181a;
Figure 14 is a graph showing the effect of the antisense PNA on the target miR23a;
Figure 15 is a graph showing the effect of the antisense PNA on the target miR19b;
Figure 16 is a graph showing the effect of the antisense PNA on the target miR20a;
Figure 17 is a graph showing the effect of the antisense PNA on the target let7g;
Figure 18 is a graph showing the effect of the antisense PNA on the target miR34a;
Figure 19 is a graph showing the effect of the antisense PNA on the target miR30a;
Figure 20 is a graph showing the effect of the antisense PNA on the target miR146a;
Figure 21 is a graph showing the effect of the antisense PNA on the target miR130a;
Figure 22 is a graph showing the effect of the antisense PNA on the target miR155;
Figure 23 is a graph showing the effect of the antisense PNA on the target miR373;
Figure 24 is a graph showing the effect of the antisense PNA on the target miR122a;
Figure 25 is a graph showing the effect of the antisense PNA on the target miR145;
Figure 26 is a graph showing the effect of the antisense PNA on the target miR191;
Figure 27 is a graph showing the effect of the antisense PNA on the target miR193b; and
Figure 28 is a graph showing the effect of the antisense PNA on the target miR802.

Thus, the present invention comprises the following embodiments:
[Item 1]
   A microRNA antisense PNA (Peptide Nucleic Acid), which consists of 10 to 25 nucleotides, and is capable of complementarily binding with microRNA, thereby inhibiting the activity or function thereof.
[Item 2]
   The antisense PNA according to item 1, wherein the microRNA is selected from the group consisting of miR16, miR221, miR222, miR31, miR24, miR21, miR181a, miR23a, miR19b, miR20a, let7g, miR34a, miR30a, miR146a, miR130a, miR155, miR373, miR122a, miR145, miR191, miR193b and miR802.
[Item 3]
   The antisense PNA according to item 2, consisting of one of nucleotide sequences represented by SEQ. ID Nos. 1 to 4, 7, 11, 19, 21, 23, 26, 29 to 32, 34 to 36, 44, 47, 48, 51, 52, 54, 55, 59, 63, 65, 66, 68 to 80, and 82.
[Item 4]
   The antisense PNA according to item 1, which is linked with a peptide.
[Item 5]
   The antisense PNA according to item 4, wherein the peptide is for enhancing the intracellular introduction of PNA.
[Item 6]
   The antisense PNA according to item 5, wherein the peptide is selected from the group consisting of octerotide, Tat peptide, NLS (Nuclear Localization Signal), cationic peptide, H region, C-myc tag sequence, PTD (Protein Transduction Domain)-4, transportan, bacterial cell membrane active peptide, NL1.1 binding tyrosine kinase receptor, NL4c binding tyrosine kinase receptor, minimal transcription activator, pAntp / penetratin, Gal 80 BP, signal-sequence based peptide (I), signal-sequence based peptide (II), ^{99m}Tc chelating peptide, IGF1, mitochondria acquired peptide, YDEGE, M918 and R₆-Pen, and those derived therefrom.
[Item 7]
   The antisense PNA according to item 6, wherein the peptide consists of the amino acid sequence represented by SEQ. ID No. 83 or 84.
[Item 8]
   A composition for inhibiting the activity or function of microRNA, containing the microRNA antisense PNA according to any one of items 1 to 7, as an active ingredient.
[Item 9]
   A method for inhibiting the activity or function of microRNA, comprising the step of introducing into cells the microRNA antisense PNA according to any one of items 1 to 7.
[Item 10]
   The method according to item 9, wherein the microRNA antisense PNA is introduced into cells by using cationic lipid.
[Item 11]
   A method for evaluating the effectiveness of microRNA antisense PNA, comprising the step of measuring and comparing the expressions of microRNA, in presence and absence of the microRNA antisense PNA.
[Item 12]
   The method according to item 11, wherein the expressions of microRNA are measured by using reporter gene, Northern blot, microarray, real time PCR, *in vivo* / in situ hybridization or labeling.
[Item 13]
   The method according to item 12, comprising the steps of:
   (a) mixing the antisense PNA with a control vector containing a reporter gene and an experimental vector containing another reporter gene and a target microRNA binding sequence, and then, introducing the mixture into cells; and
   (b) measuring and comparing the expressions from the reporter genes in the control vector and the experimental vector of step (a).
[Item 14]
   The method according to item 11, wherein the expressions of microRNA are measured after cultivating cells for 24 to 36 hours.

### [Best Mode]

Hereinafter, the present invention will be described in detail.

The present invention relates to a microRNA antisense PNA complementarily binding with microRNA, thereby inhibiting the activity or function of microRNA. The antisense PNA of the present invention consists of 10 to 25 nucleotides, particularly, 15 nucleotides. It will be appreciated that short PNA of 10 to 14mer, long PNA of 16 to 25mer, and PNA containing a part of 5' and 3' regions, corresponding to seed region, of microRNA, can also sufficiently function as microRNA antisense, and thus, all of these PNAs fall within the scope of the present invention. In this invention, the microRNA includes any kind of microRNA, without limitation; for example, miR16, miR221, miR222, miR31, miR24, miR21, miR181a, miR23a, miR19b, miR20a, let7g, miR34a, miR30a, miR146a, miR130a, miR155, miR373, miR122a, miR145, miR191, and miR193b, but not limited thereto. The nucleotide sequence of antisense PNA of the present invention is not specifically limited, as long as it can complementarily bind to microRNA to inhibit the activity or function thereof. For example, the antisense PNA consists of one of the nucleotide sequences represented by SEQ. ID Nos. 1 to 82, preferably by SEQ. ID Nos. 1 to 4, 7, 11, 19, 21, 23, 26, 29 to 32, 34 to 36, 44, 47, 48, 51, 52, 54, 55, 59, 63, 65, 66, 68 to 80, and 82, as set forth in the following Table 1, but not limited thereto.

**[Table 1]**

| SEQ. ID No | Designation | Nucleotide sequence | Description |
|---|---|---|---|
| 1 | miR16-1 | atttacgtgctgcta | Antisense to miR16 |
| 2 | miR16-2 | tatttacgtgctgct | Antisense to miR16 |
| 3 | miR16-3 | atatttacgtgctgc | Antisense to miR16 |
| 4 | miR16-4 | aatatttacgtgctg | Antisense to miR16 |
| 5 | miR16-5 | caatatttacgtgct | Antisense to miR16 |
| 6 | miR16-6 | ccaatatttacgtgc | Antisense to miR16 |
| 7 | miR16-7 | gccaatatttacgtg | Antisense to miR16 |
| 8 | miR16-8 | cgccaatatttacgt | Antisense to miR16 |
| 9 | miR16-9 | caatatttacgtgctgct | Antisense to miR16 |
| 10 | miR221-1 | gcagacaatgtagct | Antisense to miR221 |
| 11 | miR221-2 | agcagacaatgtagc | Antisense to miR221 |
| 12 | miR221-3 | cagcagacaatgtag | Antisense to miR221 |
| 13 | miR221-4 | ccagcagacaatgta | Antisense to miR221 |
| 14 | miR221-5 | cccagcagacaatgt | Antisense to miR221 |
| 15 | miR221-6 | acccagcagacaatg | Antisense to miR221 |
| 16 | miR221-7 | aacccagcagacaat | Antisense to miR221 |
| 17 | miR221-8 | aaacccagcagacaa | Antisense to miR221 |
| 18 | miR221-9 | gaaacccagcagaca | Antisense to miR221 |
| 19 | miR221-10 | cccagcagacaatgtagc | Antisense to miR221 |
| 20 | miR222-1 | tagccagatgtagct | Antisense to miR222 |
| 21 | miR222-2 | gtagccagatgtagc | Antisense to miR222 |
| 22 | miR222-3 | agtagecagatgtag | Antisense to miR222 |
| 23 | miR222-4 | cagtagccagatgta | Antisense to miR222 |
| 24 | miR222-5 | ccagtagccagatgt | Antisense to miR222 |
| 25 | miR222-6 | cccagtagccagatg | Antisense to miR222 |
| 26 | miR222-7 | acccagtagccagat | Antisense to miR222 |
| 27 | miR222-8 | gacccagtagccaga | Antisense to miR222 |
| 28 | miR222-9 | agacccagtagccag | Antisense to miR222 |
| 29 | miR222-10 | gagacccagtagcca | Antisense to miR222 |
| 30 | miR31-1 | tgccagcatcttgcc | Antisense to miR31 |
| 31 | miR31-2 | atgccagcatcttgc | Antisense to miR31 |
| 32 | miR31-3 | tatgccagcatcttg | Antisense to miR31 |
| 33 | miR31-4 | ctatgccagcatctt | Antisense to miR31 |
| 34 | miR31-5 | gctatgccagcatct | Antisense to miR31 |
| 35 | miR31-6 | agctatgccagcatc | Antisense to miR31 |
| 36 | miR31-7 | cagctatgccagcat | Antisense to miR31 |
| 37 | miR24-1 | tgctgaactgagcca | Antisense to miR24 |
| 38 | miR24-2 | ctgctgaactgagcc | Antisense to miR24 |
| 39 | miR24-3 | cctgctgaactgagc | Antisense to miR24 |
| 40 | miR24-4 | tcctgctgaactgag | Antisense to miR24 |
| 41 | miR24-5 | ttcctgctgaactga | Antisense to miR24 |
| 42 | miR24-6 | gttcctgctgaactg | Antisense to miR24 |
| 43 | miR24-7 | tgttcctgctgaact | Antisense to miR24 |
| 44 | miR24-8 | ctgttcctgctgaac | Antisense to miR24 |
| 45 | miR21-2 | cagtctgataagcta | Antisense to miR21 |
| 46 | miR21-3 | tcagtctgataagct | Antisense to miR21 |
| 47 | miR21-8 | caacatcagtctgat | Antisense to miR21 |
| 48 | miR181a-1 | gacagcgttgaatgt | Antisense to miR181a |
| 49 | miR181a-2 | tcaccgacagcgttgaatgt | Antisense to miR181a |
| 50 | miR23a-1 | tccctggcaatgtga | Antisense to miR23a |
| 51 | miR23a-2 | ggaaatccctggcaatgtga | Antisense to miR23a |
| 52 | miR19b-1 | tgcatggatttgcac | Antisense to miR19b |
| 53 | miR19b-2 | agttttgcatggatttgcac | Antisense to miR19b |
| 54 | miR20a-1 | cactataagcacttt | Antisense to miR20a |
| 55 | miR20a-2 | acctgcactataagcacttt | Antisense to miR20a |
| 56 | let7g-1 | caaactactacctca | Antisense to let7g |
| 57 | let7g-2 | acaaactactacctc | Antisense to let7g |
| 58 | let7g-3 | tacaaactactacct | Antisense to let7g |
| 59 | let7g-4 | gtacaaactactacc | Antisense to let7g |
| 60 | let7g-5 | tgtacaaactactac | Antisense to let7g |
| 61 | let7g-6 | ctgtacaaactacta | Antisense to let7g |
| 62 | let7g-7 | actgtacaaactact | Antisense to let7g |
| 63 | miR34a-1 | agctaagacactgcc | Antisense to miR34a |
| 64 | miR34a-2 | caaccagctaagacactgcc | Antisense to miR34a |
| 65 | miR30a-1 | gtcgaggatgtttac | Antisense to miR30a |
| 66 | miR146a-1 | tggaattcagttctc | Antisense to miR146a |
| 67 | miR146a-2 | ccatggaattcagttctc | Antisense to miR146a |
| 68 | miR130a-1 | ttttaacattgcact | Antisense to miR130a |
| 69 | miR130a-2 | cccttttaacattgcact | Antisense to miR130a |
| 70 | miR155-1 | tcacgattagcatta | Antisense to miR155 |
| 71 | miR155-2 | ctatcacgattagca | Antisense to miR155 |
| 72 | miR373-1 | aaaatcgaagcactt | Antisense to miR373 |
| 73 | miR373-2 | cccaaaatcgaagcactt | Antisense to miR373 |
| 74 | miR122-1 | ccattgtcacactcc | Antisense to miR122 |
| 75 | miR122-2 | acaccattgtcacactcc | Antisense to miR122 |
| 76 | miR145-1 | cctgggaaaactgga | Antisense to miR145 |
| 77 | miR145-2 | attcctgggaaaactgga | Antisense to miR145 |
| 78 | miR191-1 | ttttgggattccgtt | Antisense to miR191 |
| 79 | miR191-2 | tgcttttgggattccgtt | Antisense to miR191 |
| 80 | miR193b-1 | actttgagggccagt | Antisense to miR193b |
| 81 | miR802-1 | tgaatctttgttact | Antisense to miR802 |
| 82 | miR802-2 | ggatgaatctttgttact | Antisense to miR802 |

The PNA of the present invention can be introduced into cells, as it is, to inhibit the activity or function of microRNA. However, since PNA is electrically neutral, cellular lipids might interrupt its intracellular introduction. To overcome such problem, many studies have been conducted on its intracellular delivery system. As a result, various approaches have been known, for example, induction of cellular uptake by attaching cell penetrating protein (CPP) (Pooga M, Hallbrink M, Zorko M, and Langel U. (1998) Cell penetration by transportan. Faseb J. 12: 67-77), Insulin-like growth factor I-receptor (Basu S, and Wickstrom E. (1997) Synthesis and characterization of a peptide nucleic acid conjugated to a D-peptide analog of insulin-like growth factor 1 for increased cellular uptake. Bioconjug. Chem. 8: 481-488), or asialoglycoprotein receptor (Zhang X, Simmons C G, and Corey D R. (2001) Liver cell specific targeting of peptide nucleic acid oligomers. Bioorg Med. Chem. Lett. 11: 1269-1271); or by using electroporation (Wang G, Xu X, Pace B, Dean D A, Glazer P M, Chan P, Goodman S R, and Shokolenko I. (1999) Peptide nucleic acid (PNA) binding-mediated induction of human gamma-globin gene expression. Nucleic Acids Res. 27(13):2806-2813) or liposome (Faruqi A F, Egholm M, and Glazer P M. (1998) Peptide nucleic acid-targeted mutagenesis of a chromosomal gene in mouse cells. Proc. Natl. Acad. Sci. U S A. 95(4):1398-1403). CPP is generally classified into the following three groups. First group is Tat peptide consisting of amino acids in the position of 49 to 57 of Tat protein, which is involved in the transcription of HIV-I causing acquired immunodeficiency syndrome. Second group is penetratin, a peptide derived from homeodomain, which has been first discovered in homeodomain of antennapedia, homeoprotein of Drosophila. Third group is membrane translocating sequence (MTS) or signal sequence based peptide. Examples of peptide, which can be efficiently used for intracellular introduction of PNA, are shown in the following Table 2. Any one of them or one derived therefrom can be linked to PNA and used in the present invention.

**[Table 2]**

| Type of Peptide | Sequence |
|---|---|
| Octreotide (SMSTR binding) | _{D}F-_{C}[CF_{D}WKTC]T (D: D type, C: cyclic peptide) |
| Tat peptide | GRKKRRQRRRPPQ |
| NLS(Nuclear localization signal) | PKKKRKV |
| Cationic peptide | KKKK, or KK [AAKK]₃ or KK [SSKK]₃ |
| H region | AAVALLPAVLLALLA |
| C-myc tag sequence | EQKLISEEDLNA |
| PTD(Protein transduction domain)-4 | YARAAARQARA |
| Transportan (Designed cell membrane active peptide) | GWTLNSAGYLLGKINLKALA-ALAKKIL |
| Bacterial cell membrane active protein | KFFKFFKFFK |
| NL1.1 binding tyrosine kinase receptor | AEGEFMYWGDSHWLQYWYE-GDPAKGGSGGGSGGGKG |
| NL4c binding tyrosine kinase receptor | AEGEFFCVSSGGGSSCWPDPA-KGGSGGGSGGGSKG |
| Minimal transcription activator | GG-[PADALDDFDLDML]_{2,3} |
| Pantennapedia (43-58) pAntp/penetratin | RQIKIWFQNRRMKWKK |
| Active domain for gene-specific transcription activation (Gal 80 BP) | RHGEKWFLDDFTNNQM |
| Signal sequence based peptide (I) | QPKKKRKV |
| Signal sequence based peptide (II) | AAVALLPAVLLALLAP |
| ^{99m}Tc chelating peptide | G_{D}AGG (D: D type) |
| IGF1 | _{D}[GGGGCSKC] (D: D type) |
| Mitochondria acquired peptide | MSVLTPLLLRGLTGSARRLPVPRAKIHSL |
| YDEGE | YDEEGGGE-NH₂ |
| M918 | MVTVLFRRLRIRRACGPPRVRV-NH₂ |
| R₆-Pen | NH₂-RRRRRRRQIKIWFQNRRMKWKKGGC |

In addition, other known or novel peptides, effectively used for PNA, can be linked to PNA and used. Those peptide can be directly linked with PNA, but is preferably linked with PNA via an appropriate linker, such as 8-amino-3,6-dioxaoctanoic acid linker (O-linker), E-linker represented by the following formula 1, and X-linker represented by the following formula 2.

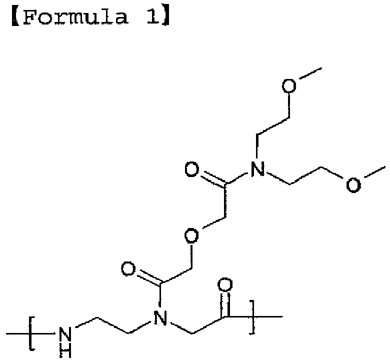

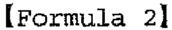

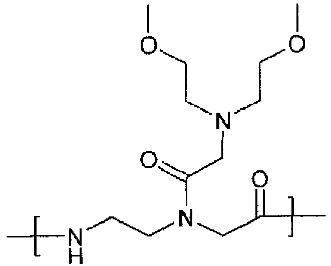

In addition to the above enumerated peptides, polyarginine, penetratin, and α-aminoacridine are known to enhance intracellular introduction of PNA. So, any of them can be linked with PNA in this invention. In one embodiment, modified Tat peptide, particularly, R peptide consisting of the amino acid sequence represented by SEQ. ID No: 83 (RRRQRRKKR), or K peptide consisting of the amino acid sequence represented by SEQ. ID No: 84 (KFFKFFKFFK) may be used to enhance intracellular introduction of PNA.

In this invention, the microRNA antisense PNA can be introduced into cells, thereby inhibiting the activity or function of microRNA. The microRNA antisense PNA can be introduced into cells by using cationic lipid, such as Lipofectamine 2000 (Invitrogen). In addition, other methods, such as electroporation or use of liposome, can be applied for intracellular introduction of the antisense PNA, and in such case, PNA with or without linked peptide may be used to act as microRNA antisense.

Further, the present invention provides a composition for inhibiting the activity or function of microRNA, containing the microRNA antisense PNA as an active ingredient. For example, the composition of the present invention can be used as a preventive or therapeutic agent for microRNA mediated diseases. The effective dose of the microRNA antisense PNA can be suitably determined by considering age, sex, health condition, type and severity of disease, etc. For example, for an adult, it may be administered at 0.1~200 mg per time, and once, twice or three times a day. For administration, any conventional gene therapy, for example, *ex vivo* or *in vivo* therapy, may be used without limitation.

In this invention, the effectiveness of the antisense PNA can be evaluated by measuring and comparing the expressions of microRNA, in presence and absence of the antisense PNA. For measuring expressions, any conventional methods known in the art can be used. For example, reporter gene, Northern blot, microarray, real time PCR, *in vivo* / *in situ* hybridization, or labeling can be used. In one embodiment, in case of measuring expressions by using report gene, the effectiveness of microRNA antisense PNA can be evaluated by the method comprising the following steps:
(a) mixing the antisense PNA with a control vector containing a reporter gene (ex: Renilla luciferase), not a target microRNA binding sequence, and an experimental vector containing another reporter gene (ex: firefly luciferase) and the target microRNA binding sequence, and then, introducing the mixture into cells; and,
(b) measuring and comparing the expressions from the reporter genes in the control vector and the experimental vector of step (a).

The experimental vector can be constructed by introducing the target microRNA binding sequence into a vector containing the reporter gene (ex: firefly luciferase).

Hereinafter, the present invention will be described in more detail with reference to the following examples, which are provided only for the better understanding of the invention, and should not be construed to limit the scope of invention in any manner.

### Example 1: Synthesis of antisense PNA

To investigate the antisense effect of PNA against microRNA, the antisense PNAs having the complementary sequences with specific target microRNAs, i.e. miR16, miR221, miR222, miR31, miR24, miR21, miR181a, miR23a, miR19b, miR20a, let7g, miR34a, miR30a, miR146a, miR130a, miR155, miR373, miR122a, miR145, miR191, miR193b and miR802, were synthesized. In general, microRNAs consist of 21 to 25 nucleotides, among which 2^{nd} to 8^{th} nucleotides are known as seed sequence.

PNAs having various sequences, for example, complementary with 1^{st} to 15^{th}, 2^{nd} to 16^{th}, or 3^{rd} to 17^{th} nucleotides of target microRNA, were synthesized so that they could complementarily bind with the target microRNA. Modified HIV-1 Tat peptide (R-peptide, RRRQRRKKR) was linked to those PNAs via O-linker. To evaluate the effect of the modified Tat peptide, antisense PNAs were also linked with K-peptide (KFFKFFKFFK), known to enhance intracellular introduction of PNA into *E*. *coli,* not into animal cells. The control PNAs (con-K, con-R and con-2R) having no antisense activity were also synthesized. The synthesized antisense PNAs and the control PNAs are shown in the following Table 3.

**[Table 3]**

| Designation | Sequence of Antisense against specific microRNA/control PNA | SEQ. ID Nos. of the nucleotide sequence of antisense/ control PNA |
|---|---|---|
| miR16-1(R) | RRRQRRKKR-O-atttacgtgctgcta | 1 |
| miR16-2(R) | RRRQRRKKR-O-tatttacgtgctgct | 2 |
| miR16-3(R) | RRRQRRKKR-O-atatttacgtgctgc | 3 |
| miR16-4(R) | RRRQRRKKR-O-aatatttacgtgctg | 4 |
| miR16-5(R) | RRRQRRKKR-O-caatatttacgtgct | 5 |
| miR16-6(R) | RRRQRRKKR-O-ccaatatttacgtgc | 6 |
| miR16-7(R) | RRRQRRKKR-O-gccaatatttacgtg | 7 |
| miR16-8(R) | RRRQRRKKR-O-cgccaatatttacgt | 8 |
| miR16-9(R) | RRRQRRKKR-O-caatatttacgtgctgct | 9 |
| miR16-2 | tatttacgtgctgct | 2 |
| miR16-1(K) | KFFKFFKFFK-O-atttacgtgctgcta | 1 |
| miRl6-2(K) | KFFKFFKFFK-O-tatttacgtgctgct | 2 |
| miR16-3(K) | KFFKFFKFFK-O-atatttacgtgctgc | 3 |
| miR16-4(K) | KFFKFFKFFK-O-aatatttacgtgctg | 4 |
| miR16-5(K) | KFFKFFKFFK-O-caatatttacgtgct | 5 |
| miR16-6(K) | KFFKFFKFFK-O-ccaatatttacgtgc | 6 |
| miR16-7(K) | KFFKFFKFFK-O-gccaatatttacgtg | 7 |
| miR16-8(K) | KFFKFFKFFK-O-cgccaatatttacgt | 8 |
| miR16-9(K) | KFFKFFKFFK-O-caatatttacgtgctgct | 9 |
| miR221-1(R) | RRRQRRKKR-O-gcagacaatgtagct | 10 |
| miR221-2(R) | RRRQRRKKR-O-agcagacaatgtagc | 11 |
| miR221-3(R) | RRRQRRKKR-O-cagcagacaatgtag | 12 |
| miR221-4(R) | RRRQRRKKR-O-ccagcagacaatgta | 13 |
| miR221-5(R) | RRRQRRKKR-O-cccagcagacaatgt | 14 |
| miR221-6(R) | RRRQRRKKR-O-acccagcagacaatg | 15 |
| miR221-7(R) | RRRQRRKKR-O-aacccagcagacaat | 16 |
| miR221-8(R) | RRRQRRKKR-O-aaacccagcagacaa | 17 |
| miR221-9(R) | RRRQRRKKR-O-gaaacccagcagaca | 18 |
| miR221-10(R) | RRRQRRKKR-O-cccagcagacaatgtagc | 19 |
| miR222-1(R) | RRRQRRKKR-O-tagccagatgtagct | 20 |
| miR222-2(R) | RRRQRRKKR-O-gtagccagatgtagc | 21 |
| miR222-3(R) | RRRQRRKKR-O-agtagccagatgtag | 22 |
| miR222-4(R) | RRRQRRKKR-O-cagtagccagatgta | 23 |
| miR222-5(R) | RRRQRRKKR-O-ccagtagccagatgt | 24 |
| miR222-6(R) | RRRQRRKKR-O-cccagtagccagatg | 25 |
| miR222-7(R) | RRRQRRKKR-O-acccagtagccagat | 26 |
| miR222-8(R) | RRRQRRKKR-O-gacccagtagccaga | 27 |
| miR222-9(R) | RRRQRRKKR-O-agacccagtagccag | 28 |
| miR222-10(R) | RRRQRRKKR-O-gagacccagtagcca | 29 |
| miR31-1R | RRRQRRKKR-O-tgccagcatcttgcc | 30 |
| miR31-2R | RRRQRRKKR-O-atgccagcatcttgc | 31 |
| miR31-3R | RRRQRRKKR-O-tatgccagcatcttg | 32 |
| miR31-4R | RRRQRRKKR-O-ctatgccagcatctt | 33 |
| miR31-5R | RRRQRRKKR-O-gctatgccagcatct | 34 |
| miR31-6R | RRRQRRKKR-O-agctatgccagcatc | 35 |
| miR31-7R | RRRQRRKKR-O-cagctatgccagcat | 36 |
| miR24-1R | RRRQRRKKR-O-tgctgaactgagcca | 37 |
| miR24-2R | RRRQRRKKR-O-ctgctgaactgagcc | 38 |
| miR24-3R | RRRQRRKKR-O-cctgctgaactgagc | 39 |
| miR24-4R | RRRQRRKKR-O-tcctgctgaactgag | 40 |
| miR24-5R | RRRQRRKKR-O-ttcctgctgaactga | 41 |
| miR24-6R | RRRQRRKKR-O-gttcctgctgaactg | 42 |
| miR24-7R | RRRQRRKKR-O-tgttcctgctgaact | 43 |
| miR24-8R | RRRQRRKKR-O-ctgttcctgctgaac | 44 |
| miR21-2R | RRRQRRKKR-O-cagtctgataagcta | 45 |
| miR21-3R | RRRQRRKKR-O-tcagtctgataagct | 46 |
| miR21-8R | RRRQRRKKR-O-caacatcagtctgat | 47 |
| miR181a-1R | RRRQRRKKR-O-gacagcgttgaatgt | 48 |
| miR181a-2R | RRRQRRKKR-O-tcaccgacagcgttgaatgt | 49 |
| miR23a-1R | RRRQRRKKR-O-tccctggcaatgtga | 50 |
| miR23a-2R | RRRQRRKKR-O-ggaaatccctggcaatgtga | 51 |
| miR19b-1R | RRRQRRKKR-O-tgcatggatttgcac | 52 |
| miR19b-2 | RRRQRRKKR-O-agttttgcatggatttgcac | 53 |
| miR20a-1R | RRRQRRKKR-O-cactataagcacttt | 54 |
| miR20a-2R | RRRQRRKKR-O-acctgcactataagcacttt | 55 |
| let7g-1R | RRRQRRKKR-O-caaactactacctca | 56 |
| let7g-2R | RRRQRRKKR-O-acaaactactacctc | 57 |
| let7g-3R | RRRQRRKKR-O-tacaaactactacct | 58 |
| let7g-4R | RRRQRRKKR-O-gtacaaactactacc | 59 |
| let7g-5R | RRRQRRKKR-O-tgtacaaactactac | 60 |
| let7g-6R | RRRQRRKKR-O-ctgtacaaactacta | 61 |
| let7g-7R | RRRQRRKKR-O-actgtacaaactact | 62 |
| miR34a-1R | RRRQRRKKR-O-agctaagacactgcc | 63 |
| miR34a-2R | RRRQRRKKR-O-caaccagctaagacactgcc | 64 |
| miR30a-1R | RRRQRRKKR-O-gtcgaggatgtttac | 65 |
| miR146a-1R | RRRQRRKKR-O-tggaattcagttctc | 66 |
| miR146a-2R | RRRQRRKKR-O-ccatggaattcagttctc | 67 |
| miR130a-1R | RRRQRRKKR-O-ttttaacattgcact | 68 |
| miR130a-2R | RRRQRRKKR-O-cccttttaacattgcact | 69 |
| miR155-1R | RRRQRRKKR-O-tcacgattagcatta | 70 |
| miR155-2R | RRRQRRKKR-O-ctatcacgattagca | 71 |
| miR373-1R | RRRQRRKKR-O-aaaatcgaagcactt | 72 |
| miR373-2R | RRRQRRKKR-O-cccaaaatcgaagcactt | 73 |
| miR122-1R | RRRQRRKKR-O-ccattgtcacactcc | 74 |
| miR122-2R | RRRQRRKKR-O-acaccattgtcacactcc | 75 |
| miR145-1R | RRRQRRKKR-O-cctgggaaaactgga | 76 |
| miR145-2R | RRRQRRKKR-O-attcctgggaaaactgga | 77 |
| miR191-1R | RRRQRRKKR-O-ttttgggattccgtt | 78 |
| miR191-2R | RRRQRRKKR-O-tgcttttgggattccgtt | 79 |
| miR193b-1R | RRRQRRKKR-O-actttgagggccagt | 80 |
| miR802-1R | RRRQRRKKR-O-tgaatctttgttact | 81 |
| miR802-2R | RRRQRRKKR-O-ggatgaatctttgttact | 82 |
| con-K | KFFKFFKFFK-O-gacaacaatgaatgt | 85 |
| con-R | RRRQRRKKR-O-gacaacaatgaatgt | 85 |
| con-2R | RRRQRRKKR-O-attaatgtcggacaa | 86 |

### Example 2: Evaluation of function of antisense PNA and effect of binding peptide thereon

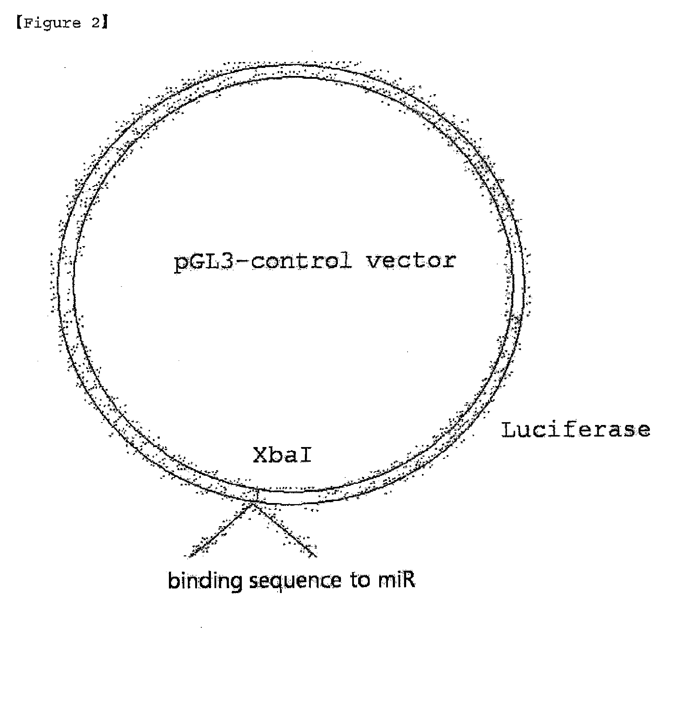

To evaluate function of the antisense PNA and effect of binding peptide thereon, HeLa cells were spread onto a 24 well plate at the density of 6×10⁴ cells / well, and cultivated for 24 hours. The cells were transformed with pGL3-control vector (Promega) having firefly luciferase gene and the cloned miR16 binding sequence (see Figure 2) and pGL3-control vector having Renilla luciferase gene, together with the antisense PNA against miR16 by using Lipofectamine 2000 (Invitrogen).

Control PNAs (con-K and con-R) were also transformed in the above manner. Expressions of reporter genes were measured to evaluate the effectiveness of the antisense PNA.

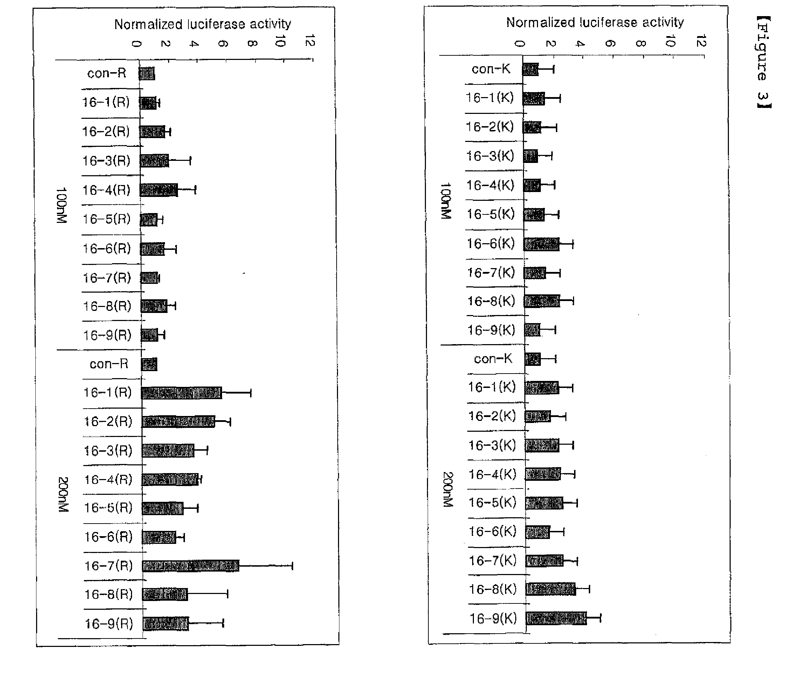

The results are shown in Figure 3. As shown in Figure 3, all the antisense PNAs of 15mer against miR16 according to this invention showed the antisense effect to inhibit function of microRNA16. It was also shown that the antisense PNAs linked with the modified Tat peptide (R peptide) had higher effects than those linked with K peptide.

### Example 3: Evaluation of the effect of linked peptide on function of antisense

To compare the effects of antisense PNAs with and without the linked modified Tat peptide, HeLa cells were spread onto a 24 well plate at the density of 6×10⁴ cells/well, and cultivated for 24 hours. The cells were transformed with pGL3-control vector (Promega) having firefly luciferase gene and the cloned miR16 binding sequence (see Figure 2) and pGL3-control vector having Renilla luciferase gene, together with 200 nM of the antisense PNA against miR16, by using Lipofectamine 2000 (Invitrogen). Control PNA (con-R) was also transformed in the above manner. After the transformation, the cells were cultivated for 48 hours. Then, the expressions of firefly luciferase and Renilla luciferase were measured by using Dual luciferase assay system (Promega).

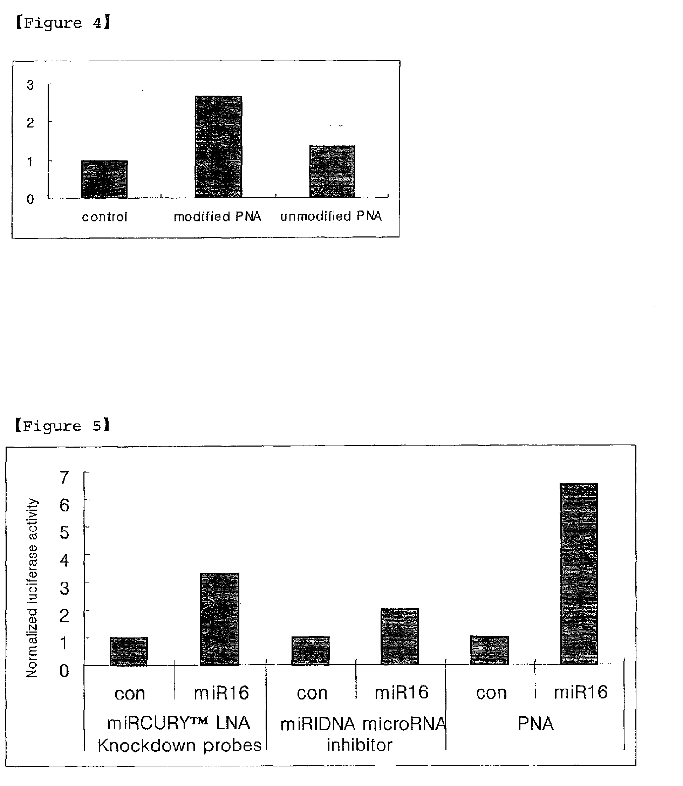

The results are shown in Figure 4. As shown in Figure 4, the antisense PNA with the modified Tat peptide (modified PNA) against miR16 showed excellent antisense effect against microRNA 16, while the PNA without the peptide (unmodified PNA, 300 nM) also showed such, but only lower, effect than the modified PNA.

### Example 4: Evaluation of the effect of PNA on target miR16

To investigate the effect of the antisense PNA against microRNA, an experimental vector containing miR16 binding sequence was used. For this, pGL3-control vector (Promega) containing firefly luciferase gene was used. To compare the level of transformation, the control vector (Promega) containing Renilla luciferase gene was used as well.

The experimental vector was constructed by inserting miR16 binding sequence into XbaI site in 3' UTR of luciferase gene of pGL-3 control vector. The sequence of miR16 was determined with reference to miR Base Sequence Database (http://microRNA.sanger.ac.uk/sequences/) (Table 4).

**[Table 4]**

| SEQ. ID No | Designation | Nucleotide sequence of microRNA |
|---|---|---|
| 87 | miR16 | UAGCAGCACGUAAAUAUUGGCG |

The corresponding complementary DNA having the same length as the microRNA was synthesized to include XbaI site in 5' and 3' regions (Table 5), and then, cloned into pGL3-control vector.

**[Table 5]**

| SEQ. ID No | Designation | miR target sequence cloning oligomer |
|---|---|---|
| 90 | miR16-F | ctagacgccaatatttacgtgctgctacgaattcaatccgt |
| 91 | miR16-R | ctagacggattgaattcgtagcagcacgtaaatattggcgt |

To compare efficiencies of the conventional microRNA antisense and the PNA antisense, miRCURY™ LNA Knockdown probe (Exiqon) against miR16 and miRIDNA (Dharmacon) against miR16 were purchased, and their effects were compared at the concentration of 200 nM. For the antisense PNA, each 100 nM of 2 kinds (#1 and #7) of PNA, which had been shown to have high efficiency at the concentration of 200 nM, as shown in Figure 3, were mixed together, and the mixture was used.

HeLa cells were cultivated for 24 hours, and transformed with the experimental vector containing miR16 binding sequence and the control vector containing Renilla luciferase gene, together with the microRNA antisense PNA, miRCURY™ LNA Knockdown probe (Exiqon) against miR16, or miRIDNA (Dharmacon) against miR16, by using Lipofectamine 2000 (Invitrogen). To confirm the microRNA inhibitory effect, the control PNA (con-R), miRCURY™ LNA Knockdown probe (Exiqon) against miRNA181b and miRIDNA (Dharmacon) against miRNA181b having the sequences not complementary with that of miR16 were also transformed in the above manner. After the transformation, the cells were cultivated for 48 hours. Then, the expressions of firefly luciferase and Renilla luciferase were measured by using Dual luciferase assay system (Promega). The results are shown in Figure 5. For the antisense PNA against miR16, the result is relative to that of the control PNA (con-R). For the miRCURY™ LNA Knockdown probe against miR16 and the miRIDNA against miR16, the results are relative to that of each one against miRNA181b. As shown in Figure 5, the antisense PNA showed 2.5 fold or more higher antisense activity against microRNA 16 than the miRCURY™ LNA Knockdown probe and the miRIDNA.

### Example 5: Evaluation of effect of miR16 antisense PNA at various concentrations

To investigate the effect of the antisense PNA against microRNA 16 at its various concentrations, HeLa cells were cultivated for 24 hours. The cells were transformed with the experimental vector containing the inserted miR16 binding sequence and the control vector containing Renilla luciferase gene, together with various concentrations (50, 100, 200 and 300 nM, respectively) of the antisense PNA (mixture of #1 and #7), by using Lipofectamine 2000 (Invitrogen). The control PNA (conR) was also transformed in the above manner. After the transformation, the cells were cultivated for 48 hours. Then, the expressions of firefly luciferase and Renilla luciferase were measured by using Dual luciferase assay system (Promega).

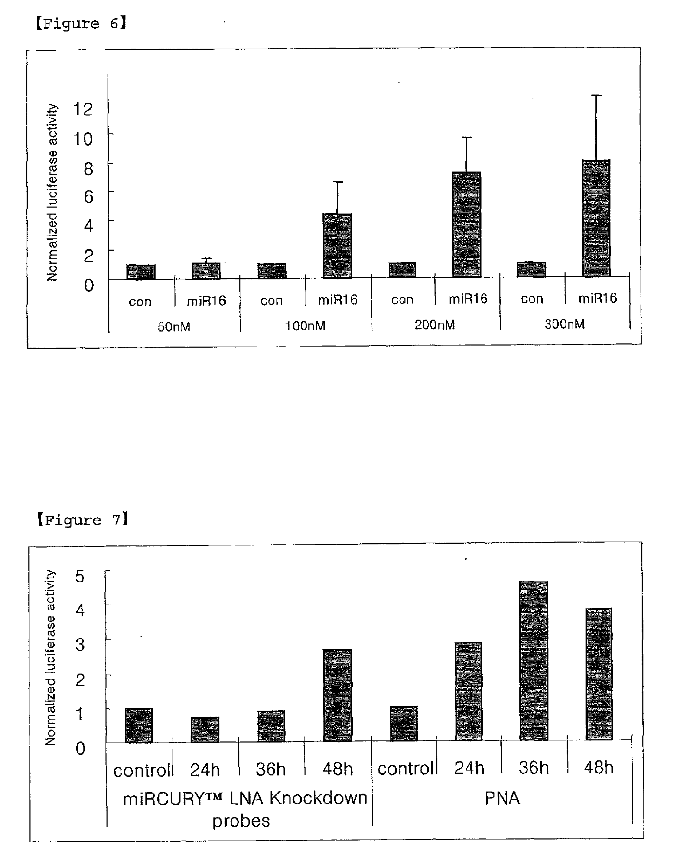

The results are shown in Figure 6. As shown in Figure 6, the highest antisense effect against miR16 could be obtained with 200 nM or more of the miR16 antisense PNA.

### Example 6: Evaluation of effect of antisense PNA against miR16 with the lapse of time

To investigate the effect of the antisense PNA against microRNA 16 with the lapse of time, HeLa cells were cultivated for 24 hours. Then, the cells were transformed with the experimental vector containing the inserted miR16 binding sequence and the control vector containing Renilla luciferase gene, together with 200 nM of the antisense PNA against miR16 (mixture of 100 nM of miR16-1 and 100 nM of miR16-7) and 200 nM of miRCURY™ LNA Knockdown probe against miR16, by using Lipofectamine 2000 (Invitrogen). To confirm the effect of the microRNA inhibitory effect, the control PNA (con-R) and miRCURY™ LNA Knockdown probe (Exiqon) against miRNA181b having the nucleotide sequence not complementary with that of miR16 were also transformed in the above manner. After the transformation, the cells were cultivated for 24, 36 and 48 hours, respectively. Then, the expressions of firefly luciferase and Renilla luciferase were measured by using Dual luciferase assay system (Promega).

The results are shown in Figure 7. For the antisense PNA against miR16, the results are relative to that of the control PNA (con-R). For the miRCURY™ LNA Knockdown probe against miR16, the results are relative to that of the probe against miRNA181b (Exiqon). As shown in Figure 7, after 12 hours, the antisense PNA showed the effect as high as that of the miRCURY™ LNA Knockdown probe after 48 hours, and after 36 hours, it showed a further increased effect, while the miRCURY™ LNA Knockdown probe showed its effect only after 48 hours. Therefore, the microRNA antisense PNA of the present invention shows the desired effect within a half period of time, as compared with the conventional microRNA antisense probe, and so it could reduce the time required for research and development.

### Example 7: Evaluation of the effect of PNA on target miR221

To construct a vector having miR221 binding sequence, a modified pGL3-control vector was used. Specifically, a synthetic oligomer containing EcoRI restriction site in 5' region and PstI restriction site in 3' region was cloned into its EcoRI/ PstI site (see Tables 6 and 7).

**[Table 6]**

| SEQ. ID No | Designation | Nucleotide sequence of microRNA |
|---|---|---|
| 88 | miR221 | AGCUACAUUGUCUGCUGGGUUUC |

**[Table 7]**

| SEQ. ID No | Designation | miR target sequence cloning oligomer |
|---|---|---|
| 92 | miR221-F | aattcgaaacccagcagacaatgtagctctgca |
| 93 | miR221-R | gagctacattgtctgctgggtttcg |

To compare the PNA antisense with the conventional antisense against microRNA, miRCURY™ LNA Knockdown probe (Exiqon) was used as well. HeLa cells were cultivated for 24 hours, and transformed with the experimental vector containing miR221 binding sequence and the control vector containing Renilla luciferase gene, together with 200 nM of the antisense PNA against miR221 and 200 nM of miRCURY™ LNA Knockdown probe (Exiqon) against miR221, by using Lipofectamine 2000 (Invitrogen). To confirm the microRNA inhibitory effect, the control PNA (con-R) and miRCURY™ LNA Knockdown probe (Exiqon) against miRNA181b having the nucleotide sequence not complementary with that of miR221 were also transformed in the above manner. After the transformation, the cells were cultivated for 48 hours. Then, the expressions of firefly luciferase and Renilla luciferase were measured by using Dual luciferase assay system (Promega).

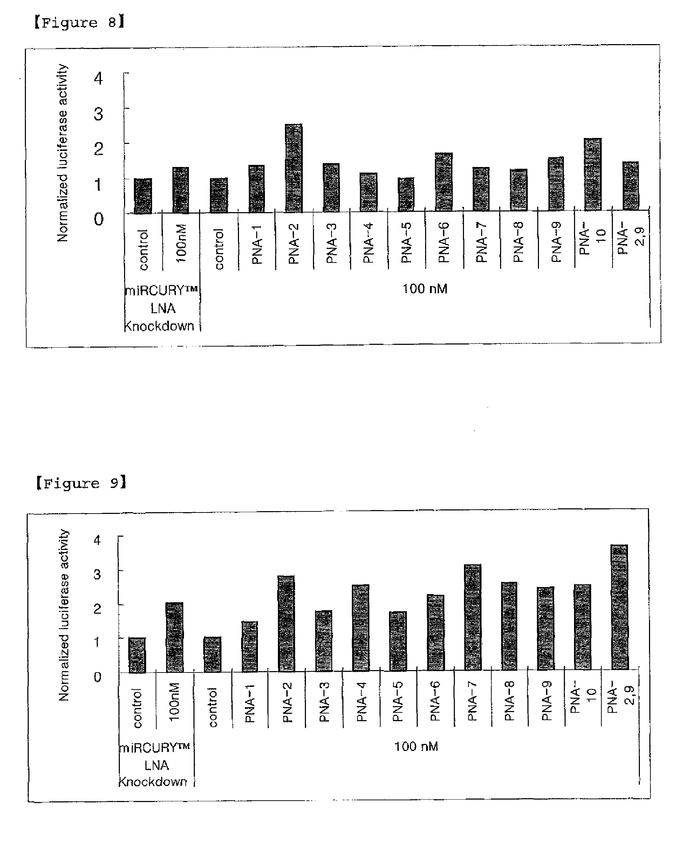

The results are shown in Figure 8. For the antisense PNA against miR221, the results are relative to that of the control PNA (con-R). For the miRCURY™ LNA Knockdown probe against miR221, the result is relative to that of the probe against miRNA181b (Exiqon). As shown in Figure 8, the miR221 antisense PNA showed a much higher antisense effect to inhibit microRNA 221 than the miRCURY™ LNA Knockdown probe.

### Example 8: Evaluation of the effect of PNA against target miR222

To construct a vector having miR222 binding sequence, a modified pGL3-control vector was used. Specifically, a synthetic oligomer containing EcoRI restriction site in 5' region and PstI restriction site in 3' region was cloned into its EcoRI/ PstI site (see Tables 8 and 9).

**[Table 8]**

| SEQ. ID No. | Designation | Nucleotide sequence of microRNA |
|---|---|---|
| 89 | miR222 | AGCUACAUCUGGCUACUGGGUCUC |

**[Table 9]**

| SEQ. ID Nos. | Designation | miR target sequence cloning oligomer |
|---|---|---|
| 94 | miR222-F | aattcgagacccagtagccagatgtagctctgca |
| 95 | miR222-R | gagctacatctggctactgggtctcg |

To compare the PNA antisense with the conventional antisense against microRNA, miRCURY™ LNA Knockdown probe (Exiqon) was used. HeLa cells were cultivated for 24 hours, and transformed with the experimental vector containing miR222 binding sequence and the control vector containing Renilla luciferase gene together with 200 nM of the antisense PNA against miR222 and 200 nM of miRCURY™ LNA Knockdown probe (Exiqon) against miR222, by using Lipofectamine 2000 (Invitrogen). To confirm the microRNA inhibitory effect, the control PNA (con-R) and miRCURY™ LNA Knockdown probe (Exiqon) against miRNA181b having the nucleotide sequence not complementary with that of miR222 were also transformed in the above manner. After the transformation, the cells were cultivated for 48 hours. Then, the expressions of firefly luciferase and Renilla luciferase were measured by using Dual luciferase assay system (Promega).

The results are shown in Figure 9. For the antisense PNA against miR222, the results are relative to that of the control PNA (con-R). For the miRCURY™ LNA Knockdown probe against miR222, the result is relative to that of the probe against miRNA181b. As shown in Figure 9, the miR222 antisense PNA showed a much higher antisense effect to inhibit microRNA 222 than the miRCURY™ LNA Knockdown probe.

### Example 9: Evaluation of effects of PNAs on targets miR31, miR24, miR21, miR181a, miR23a, miR19b, miR20a, let7g, miR34a, miR30a, miR146a, miR130a, miR155, miR373, miR122a, miR145, miR191, miR193b, and miR802

Each DNA with the same length as and complementary with miR31, miR24, miR21, miR181a, miR23a, miR19b, miR20a, let7g, miR34a, miR30a, miR146a, miR130a, miR155, miR373, miR122a, miR145, miR191, miR193b and miR802 was cloned into pGL3-control vector, according the same procedures as described in Example 3.

HeLa cells were cultivated for 24 hours, and transformed with the experimental vector containing each microRNA binding sequence and the control vector containing Renilla luciferase gene, together with 200 nM of each microRNA antisense PNA, by using Lipofectamine 2000 (Invitrogen). To confirm the microRNA inhibitory effect, the control PNA (con-2R) having the nucleotide sequence complementary with none of the microRNAs was also transformed in the above manner. After the transformation, the cells were cultivated for 48 hours. Then, the expressions of firefly luciferase and Renilla luciferase were measured by using Dual luciferase assay system (Promega).

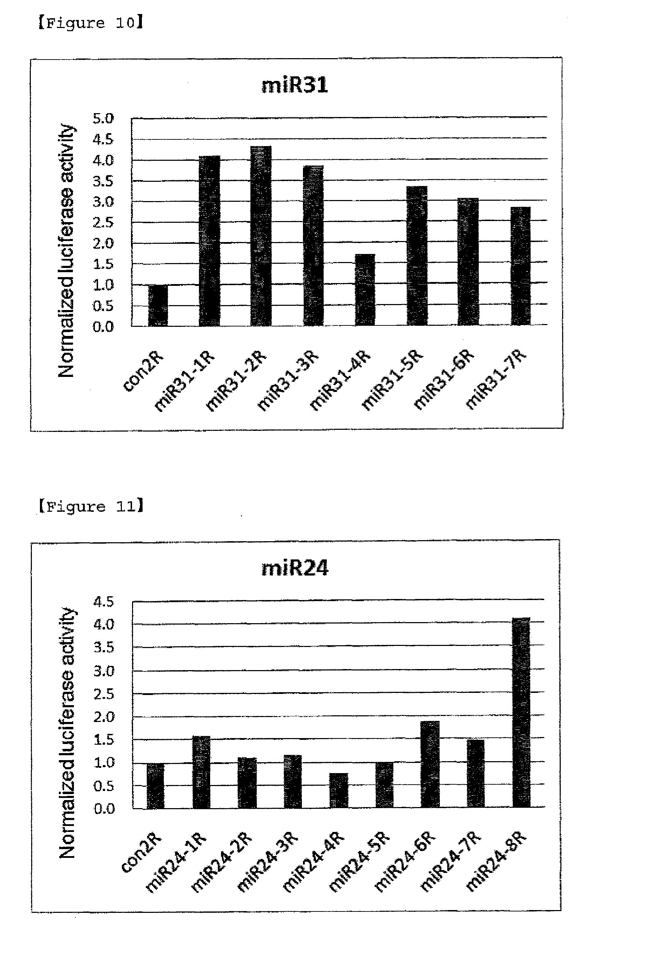

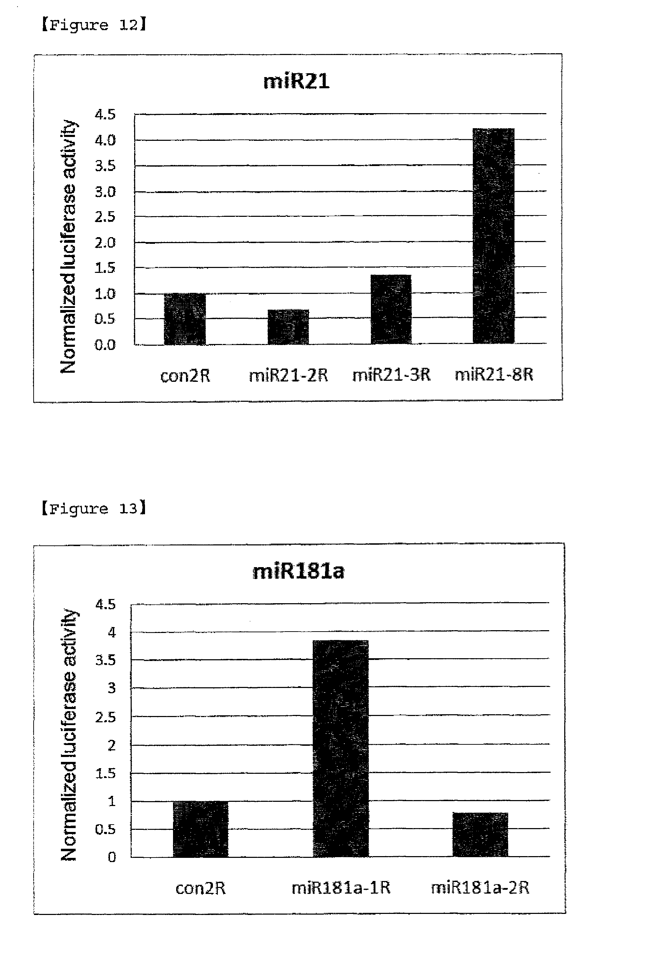

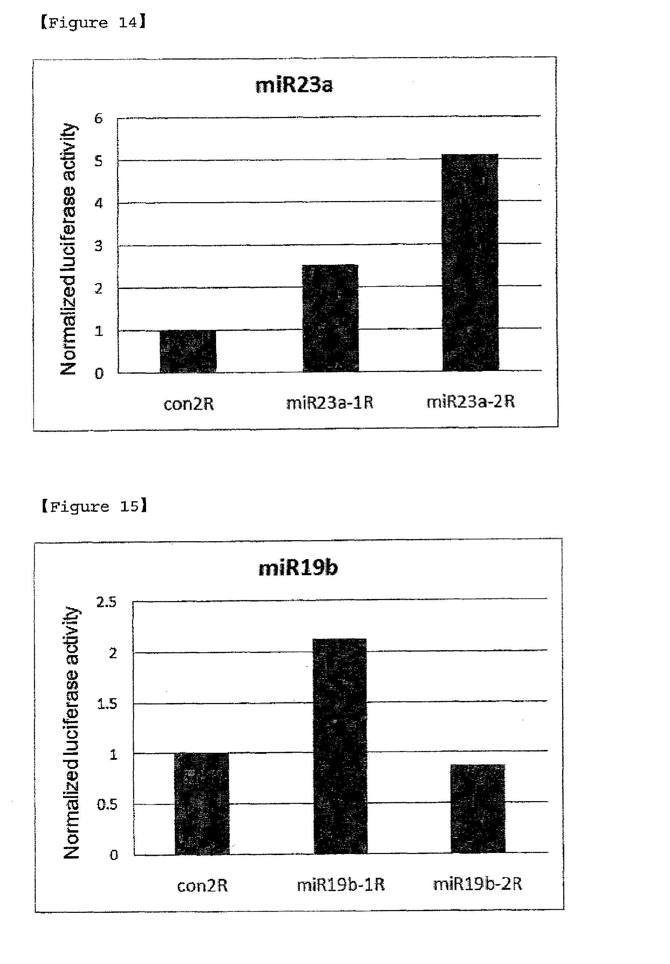

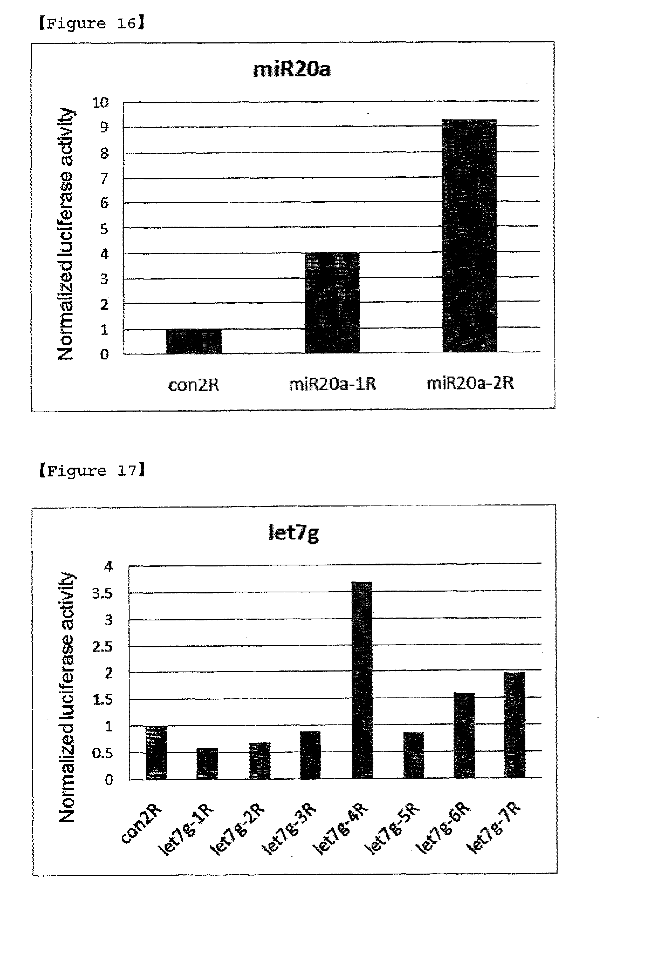

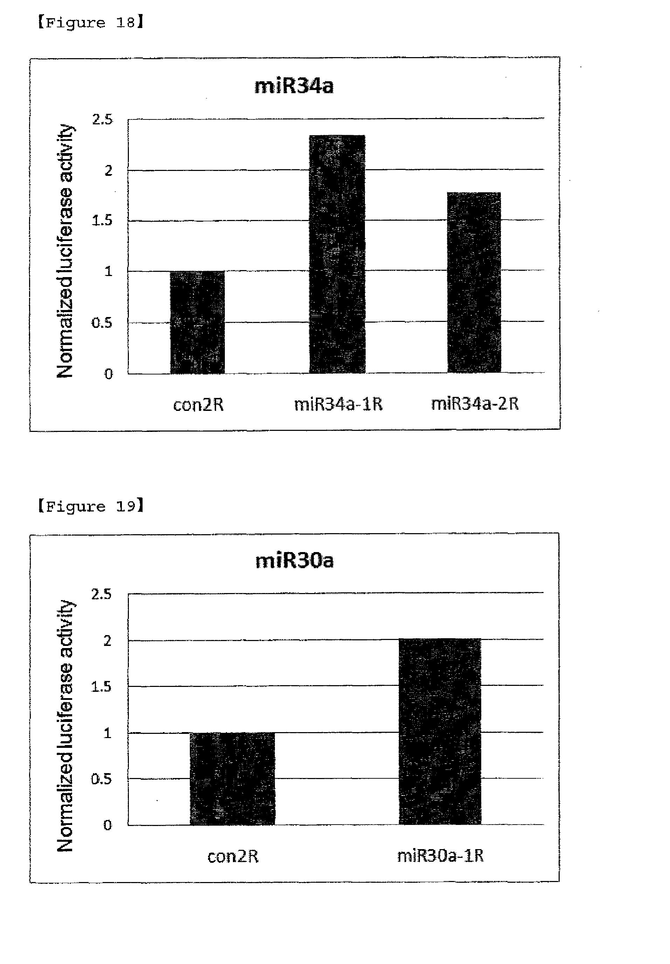

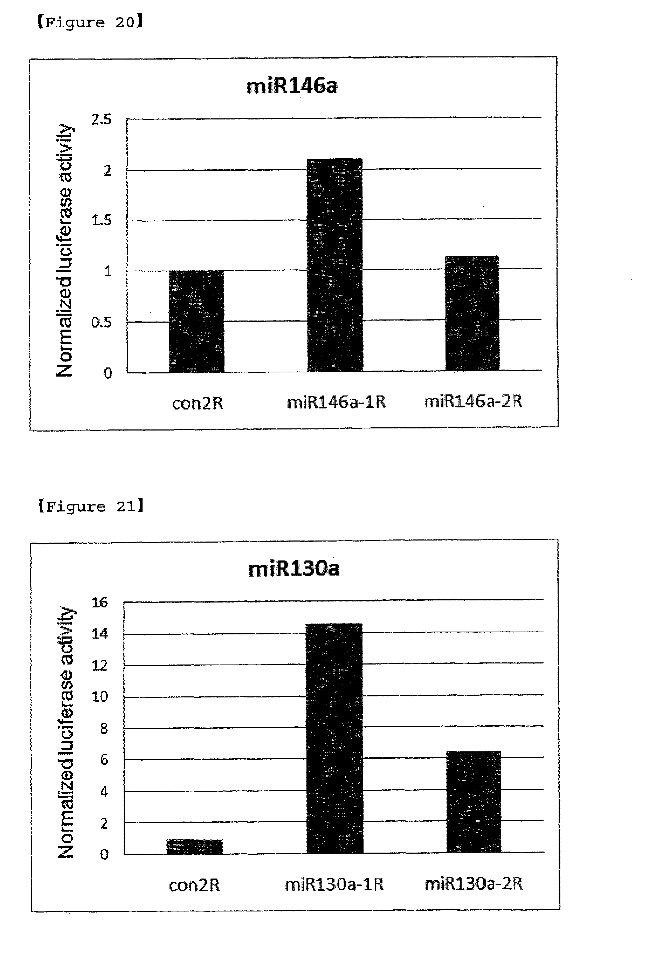

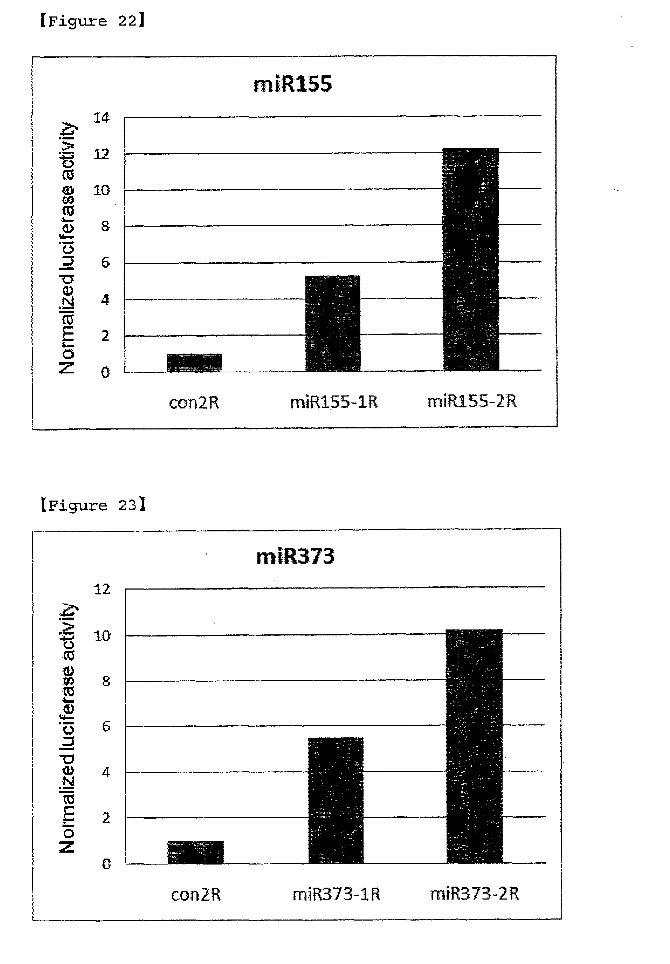

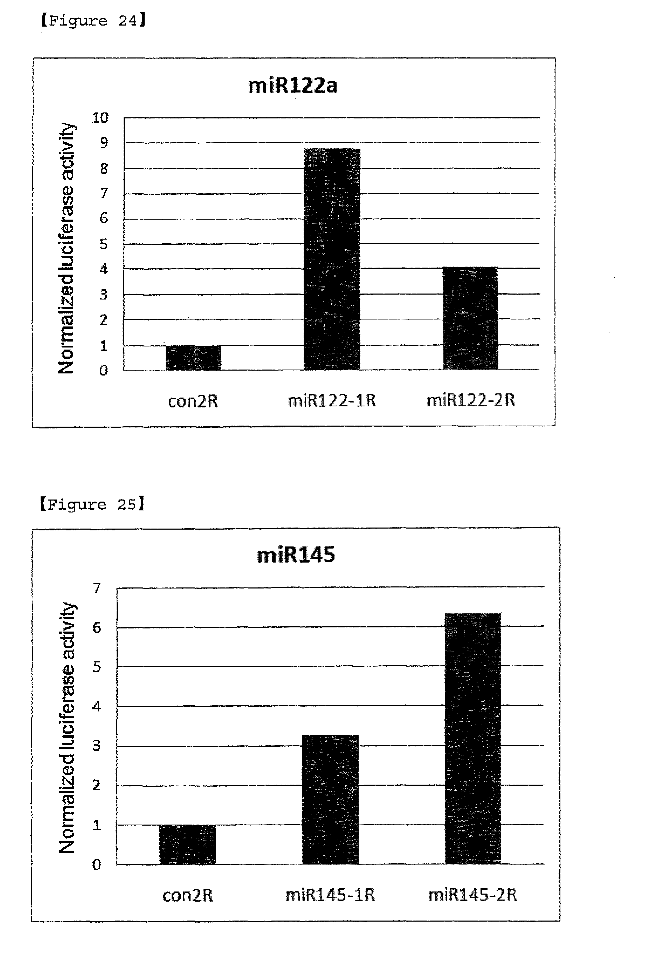

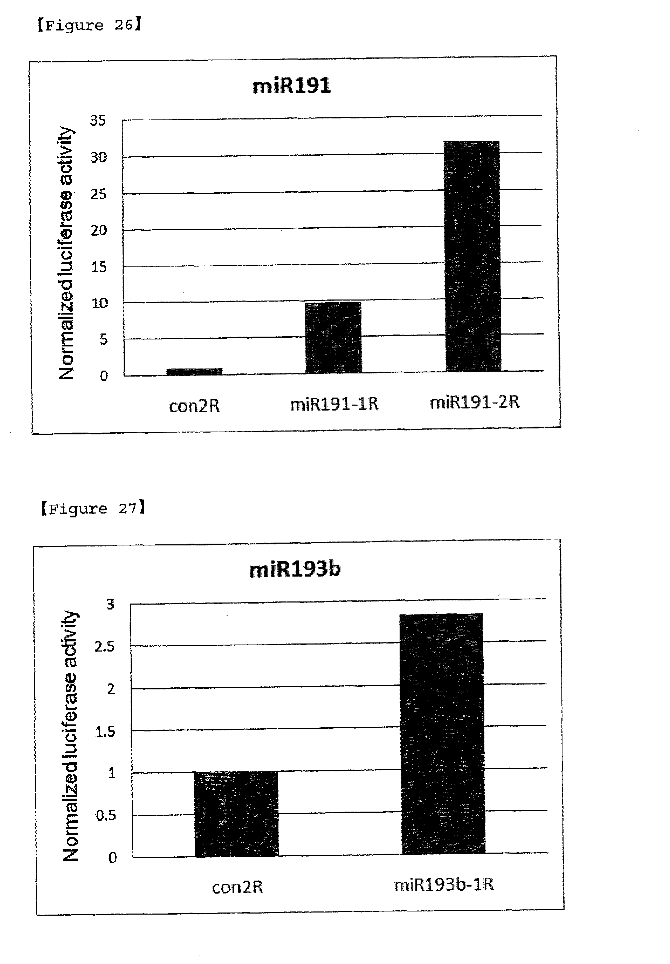

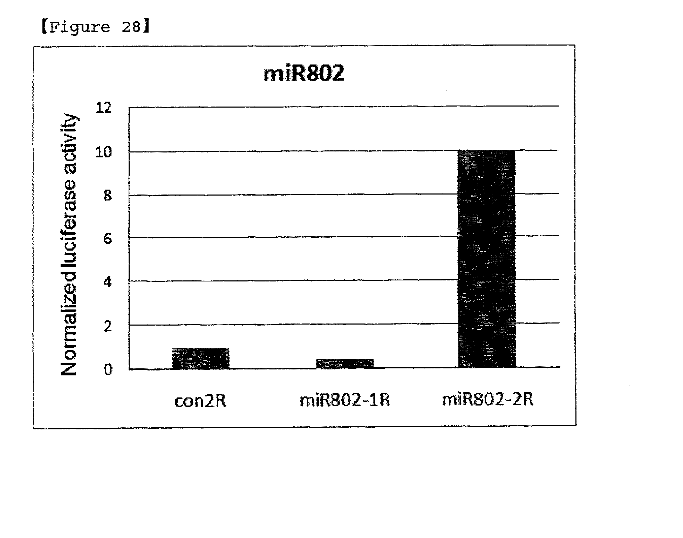

The results are shown in Figures 10 to 28. The results are relative to that of the control PNA (con-2R). As shown in the Figures, miR31-1R, miR31-2R, miR31-3R, miR31-5R, miR31-6R, miR31-7R, miR24-8R, miR21-8R, miR181-1R, miR23a-2R, miR19b-1R, miR20a-1R, miR20a-2R, let7g-4R, miR34a-1R, miR30a-1R, miR146a-1R, miR130a-1R, miR130a-2R, miR155-1R, miR155-2R, miR373-1R, miR373-2R, miR122-1R, miR122-2R, miR145-1R, miR145-2R, miR191-1R, miR191-2R, miR193b-1R, and miR802-2R antisense PNAs showed two or more fold higher miRNA inhibitory effect than the control PNA.

### [Industrial Applicability]

The microRNA antisense PNA of the present invention, an artificially synthesized DNA analogue, which can complementarily bind with DNA or RNA with a higher strength, specificity and sensitivity than DNA or RNA itself, and has high stability against not only biological degradative enzymes, such as nucleases and proteases, but also physicochemical factors, such as pH and heat, shows higher and more sustained effect in cells, and can be stored for a longer period of time, than the conventional antisense DNA or RNA. The antisense PNA of the present invention could be applied in studies for functions of microRNA to understand the regulation of gene expression in eukaryotes, and for microRNA metabolic or functional defect mediated diseases, and used as novel therapeutic agents for such diseases.

### [Sequence List Text]

SEQ. ID Nos. 1 to 82 show the nucleotide sequences of miRNA antisense PNAs;
SEQ. ID No. 83 shows the amino acid sequence of R peptide;
SEQ. ID No. 84 shows the amino acid sequence of K peptide;
SEQ. ID Nos. 85 and 86 show the nucleotide sequences of control PNAs;
SEQ. ID Nos. 87 to 89 show the nucleotide sequences of miRNAs; and
SEQ. ID Nos. 90 to 95 show the nucleotide sequences of miRNA target sequence cloning oligomers.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended claims.

## Claims

1. A microRNA antisense PNA (Peptide Nucleic Acid), which consists of 10 to 25 nucleotides, and is capable of complementarily binding with microRNA, thereby inhibiting the activity or function thereof.

2. The antisense PNA according to claim 1, wherein the microRNA is selected from the group consisting of miR21, miR221, miR222, miR31, miR24, miR181a, miR23a, miR19b, miR20a, let7g, miR34a, miR30a, miR146a, miR130a, miR155, miR373, miR122a, miR145, miR191, miR193b and miR802.

3. The antisense PNA according to claim 2, consisting of one of nucleotide sequences represented by SEQ. ID Nos. 11, 19, 21, 23, 26, 29 to 32, 34 to 36, 44, 47, 48, 51, 52, 54, 55, 59, 63, 65, 66, 68 to 80, and 82.

4. The antisense PNA according to claim 1, which is linked with a peptide.

5. The antisense PNA according to claim 4, wherein the peptide is for enhancing the intracellular introduction of PNA.

6. The antisense PNA according to claim 5, wherein the peptide is selected from the group consisting of octerotide, Tat peptide, NLS (Nuclear Localization Signal), cationic peptide, H region, C-myc tag sequence, PTD (Protein Transduction Domain)-4, transportan, bacterial cell membrane active peptide, NL1.1 binding tyrosine kinase receptor, NL4c binding tyrosine kinase receptor, minimal transcription activator, pAntp / penetratin, Gal 80 BP, signal-sequence based peptide (I), signal-sequence based peptide (II), ^{99m}Tc chelating peptide, IGF1, mitochondria acquired peptide, YDEGE, M918 and R₆-Pen, and those derived therefrom.

7. The antisense PNA according to claim 6, wherein the peptide consists of the amino acid sequence represented by SEQ. ID No. 83 or 84.

8. A composition for inhibiting the activity or function of microRNA, containing the microRNA antisense PNA according to any one of claims 1 to 7, as an active ingredient.

9. A method for inhibiting the activity or function of microRNA, comprising the step of introducing into cells the microRNA antisense PNA according to any one of claims 1 to 7.

10. The method according to claim 9, wherein the microRNA antisense PNA is introduced into cells by using cationic lipid.

11. A method for evaluating the effectiveness of microRNA antisense PNA, comprising the step of measuring and comparing the expressions of microRNA, in presence and absence of the microRNA antisense PNA.

12. The method according to claim 11, wherein the expressions of microRNA are measured by using reporter gene, Northern blot, microarray, real time PCR, *in vivo* / *in situ* hybridization or labeling.

13. The method according to claim 12, comprising the steps of:
(a) mixing the antisense PNA with a control vector containing a reporter gene and an experimental vector containing another reporter gene and a target microRNA binding sequence, and then, introducing the mixture into cells; and
(b) measuring and comparing the expressions from the reporter genes in the control vector and the experimental vector of step (a).

14. The method according to claim 11, wherein the expressions of microRNA are measured after cultivating cells for 24 to 36 hours.
